# EUROPEAN PATENT APPLICATION

(11) **EP 4 317 163 A1**
(43) Date of publication of application: **07.02.2024**
(21) Application number: 22775763.0
(22) Date of filing: 24.03.2022
(51) Int. Cl.: C07D 495/04, A61K 31/4188, A61P 3/10, A61P 17/00

(54) **METHOD FOR PREPARING BIOTIN, L-LYSINE SALT OF BIOTIN, AND METHOD FOR PREPARING SAME**

(30) Priority: 25.03.2021 JP 2021051771
(71) Applicant: Tokuyama Corporation, Shunan-shi, Yamaguchi 745-8648 (JP)
(72) Inventor: TAKAI Risa, Shunan-shi, Yamaguchi 745-8648 (JP); MATSUURA Keisuke, Shunan-shi, Yamaguchi 745-8648 (JP)
(74) Representative: Hoffmann Eitle
(86) International application number: PCT/JP2022/013894
(87) International publication number: WO 2022/202982

(57) **Abstract**

An object of the present invention is to provide a production method of biotin, and an L-lysine salt of biotin and its production method, which can efficiently remove epibiotin from the product, increase the biotin purity, and obtain biotin having a high purity, and the prevent invention provides:
a method for producing an L-lysine salt of biotin, including the following steps of: (a) bringing crude biotin into contact with L-lysine in a solvent to obtain a solution or suspension of an L-lysine salt of biotin; and (b) when the solution of the L-lysine salt of biotin is obtained in step (a), precipitating the L-lysine salt of biotin from the solution of the L-lysine salt of biotin; and
a method for producing biotin, including a step of obtaining the biotin by producing an L-lysine salt of biotin by producing an L-lysine salt of biotin by the above-described production method, and then bringing the L-lysine salt of biotin into contact with an acid.

## Description

### FIELD OF THE INVENTION

The present invention relates to a production method of biotin, and an L-lysine salt of biotin and its production method.

### BACKGROUND ART

Biotin is a water-soluble vitamin belonging to the vitamin B family (Non-Patent Document 1). Biotin is expected to have a diabetes preventive effect, a skin disease improving effect, a biotin deficiency improving effect, and the like. There is an increasing demand for biotin as a pharmaceutical, a feed additive, and the like. Biotin is represented by the following formula (1), and is also called D-biotin.

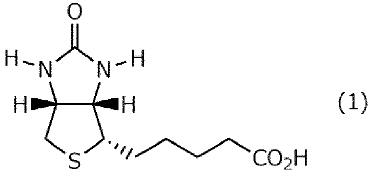

As shown below, biotin (BIF) is synthesized, for example, from a ureide (URD) via seven steps (Patent Document 1).

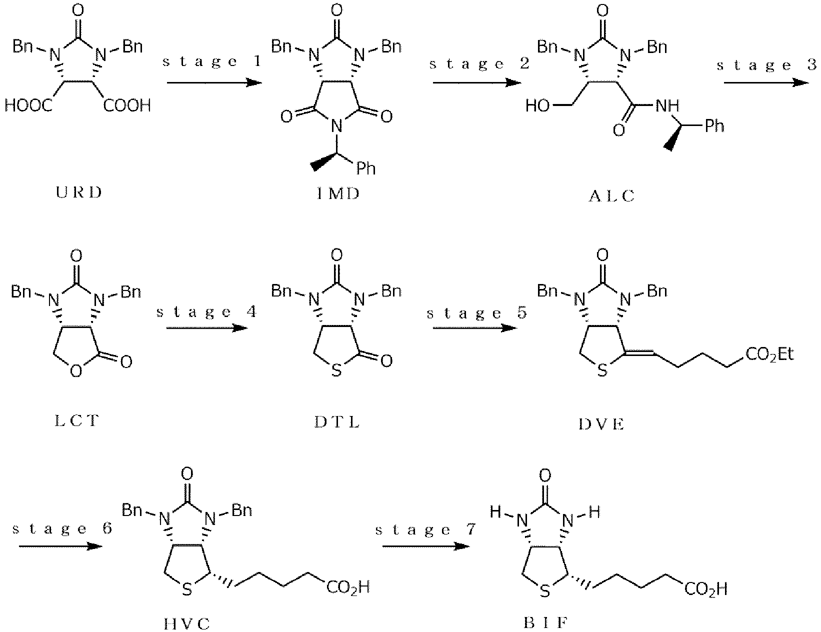

Biotin has seven isomers in addition to D-biotin represented by the above formula (1) (Non-Patent Document 2). In the process of synthesizing D-biotin, for example, isomers such as L-biotin represented by the following formula (2) may be included in the product. From a safety perspective, it is desirable to remove these isomers other than D-biotin from the product.

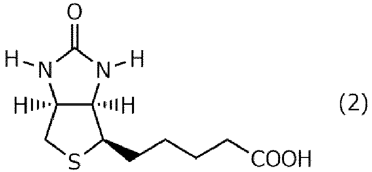

There has been disclosed a method of increasing the purity of D-biotin by reacting a racemic form of D-biotin and L-biotin with L(+)-arginine and purifying by crystallizing the resultant product as a poorly-soluble salt. However, there is no description about separation and removal of epibiotin, which is a diastereomer of biotin (Non-Patent Document 3).

### CITATION LIST

### PATENT DOCUMENT

Patent Document 1: International Publication No. 2018/025722

### NON-PATENT DOCUMENT

Non-Patent Document 1: Pierre J. de Clercq, "Biotin: A Timeless Challenge for Total Synthesis" Chemical Reviews, 1997, Vol. 97, No. 6.
Non-Patent Document 2: Toru YAMANO, Isao AOKI, and Kunio TAKANOHASHI "Direct Optical Resolution of (±)-Biotin and (±)-Epibiotin by a Reserved-Phase High-Performance Liquid Chromatography" J. Nutr. Sci. Vitaminol., 39, 419-423, 1993.
Non-Patent Document 3: DONALD E. WOLF, RALPH MOZINGO, STANTON A. HARRIS, R. CHRISTIAN ANDERSON AND KARL FOLKERS "Biotin. VI. Resolution of dl-Biotin" CONTRIBUTION FROM THE RESEARCH LABORATORIES, Journal of the American Chemical Society, 1945, Vol. 67, No. 12, 2100-2102

### SUMMARY OF INVENTION

When synthesizing biotin via the pathway as described above, the biotin-containing product may contain diastereomers and the like as impurities. Among diastereomers, epibiotin represented by the following formula (3) is an impurity that is particularly difficult to remove from the product.

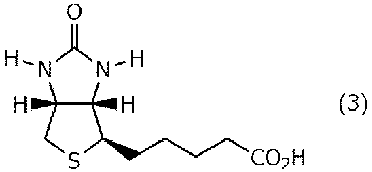

Since the area percentage of epibiotin in the product by high performance liquid chromatography (HPLC) can be as high as about 5%, epibiotin can become a factor in reducing the biotin purity. Epibiotin can be removed by, for example, repeating a purification treatment such as a neutralization crystallization treatment and an activated carbon treatment. However, a problem in that the biotin yield is reduced may arise. Therefore, a method for efficiently removing epibiotin from the product and increasing the biotin purity is desired.

Accordingly, it is an object of the present invention to provide a production method of biotin, which can efficiently remove epibiotin from the product, increase the biotin purity, and obtain high-purity biotin. Another object of the present invention is to provide an L-lysine salt of biotin and its production method, which can efficiently remove epibiotin from the product, increase the biotin purity, and obtain high-purity biotin.

As a result of intensive research by the present inventors, the inventors discovered that high-purity biotin can be obtained by bringing crude biotin containing biotin and epibiotin into contact with L-lysine in a solvent to obtain an L-lysine salt of biotin, and then perform a desalting treatment on the L-lysine salt of biotin by bringing the L-lysine salt of biotin into contact with an acid.

The present invention encompasses the following inventions.
[1] A method for producing an L-lysine salt of biotin, including the following steps of:
   (a) bringing crude biotin into contact with L-lysine in a solvent to obtain a solution or suspension of an L-lysine salt of biotin; and
   (b) when the solution of the L-lysine salt of biotin is obtained in step (a), precipitating the L-lysine salt of biotin from the solution of the L-lysine salt of biotin.
[2] The method for producing an L-lysine salt of biotin according to [1], wherein the solvent contains at least one selected from the group consisting of water, methanol, ethanol, 2-propanol, acetone and acetonitrile.
[3] The method for producing an L-lysine salt of biotin according to [1] or [2], wherein step (a) is a step of bringing crude biotin into contact with L-lysine in water to obtain a solution of an L-lysine salt of biotin.
[4] The method for producing an L-lysine salt of biotin according to any one of [1] to [3], wherein step (b) is a step of precipitating the L-lysine salt of biotin by bringing the solution of the L-lysine of biotin into contact with a precipitation solvent.
[5] The method for producing an L-lysine salt of biotin according to [4], wherein the precipitation solvent contains at least one selected from the group consisting of methanol, ethanol, 2-propanol, acetone and acetonitrile.
[6] The method for producing an L-lysine salt of biotin according to [4] or [5], wherein a volume ratio of the precipitation solvent to water (precipitation solvent/water) is 5 or more and 30 or less.
[7] The method for producing an L-lysine salt of biotin according to any one of [4] to [6], wherein step (b) is a step of precipitating the L-lysine salt of biotin by heating the solution of the L-lysine salt of biotin to 40°C or higher and 80°C or lower, then bringing the solution of the L-lysine salt of biotin into contact with the precipitation solvent, and then cooling the resulting mixture to -10°C or higher and 10°C or lower.
[8] The method for producing an L-lysine salt of biotin according to [1] or [2], wherein step (a) is a step of bringing crude biotin into contact with L-lysine in an organic solvent containing at least one selected from the group consisting of alcohol, acetone and acetonitrile to obtain a suspension of an L-lysine salt of biotin.
[9] The method for producing an L-lysine salt of biotin according to [8], wherein the organic solvent is alcohol.
[10] The method for producing an L-lysine salt of biotin according to [8] or [9], wherein an amount of the organic solvent used is 8 mL or more with respect to 1 g of the crude biotin.
[11] A method for producing biotin, including a step of producing an L-lysine salt of biotin by the method according to any one of [1] to [10], and then bringing the L-lysine salt of biotin into contact with an acid, to obtain biotin.
[12] An L-lysine salt of biotin.
[13] The L-lysine salt of biotin according to [12], wherein a content of epibiotin in terms of HPLC area percentage is 0.035% or less.

According to the method for producing an L-lysine salt of biotin according to the present invention, epibiotin can be efficiently removed from crude biotin to obtain a high-purity L-lysine salt of biotin, and by using the obtained L-lysine salt of biotin, high-purity biotin can be produced. In addition, in the method for producing biotin according to the present invention, a high-purity L-lysine salt of biotin obtained by the method for producing an L-lysine salt of biotin according to the present invention is used for producing biotin, and thus high-purity biotin can be obtained. Therefore, high-purity biotin can be obtained in a high yield without performing a purification treatment on the product at a plurality of times. Since the biotin thus obtained has a low epibiotin content, it is possible to achieve an ultra-high biotin purity, for example, a biotin content (purity) of 99.96% or more in terms of HPLC area percentage. In the present invention, the "content in terms of HPLC area percentage" is measured according to the method described in the Examples, and the HPLC is performed under the conditions described in the Examples.

Since biotin and epibiotin are weak acids, an L-lysine salt of biotin and an L-lysine salt of epibiotin can be formed by bringing biotin and epibiotin into contact with L-lysine, which has a high basicity. The L-lysine salt of biotin and the L-lysine salt of epibiotin have different solubilities in specific solvents. The magnitude of this difference in solubility is greater than the difference in solubility in specific solvents between other basic salts of biotin and basic salts of epibiotin. Therefore, by bringing the L-lysine salt of biotin and the L-lysine salt of epibiotin into contact with a solvent in which one of the salts has a high solubility and the other salt has a low solubility, a solution of the salt with the high solubility and a solid of the salt with the low solubility can be obtained. This enables the L-lysine salt of biotin and the L-lysine salt of epibiotin to be separated more efficiently. Biotin can be isolated by bringing the separated L-lysine salt of biotin into contact with an acid.

### DETAILED DESCRIPTION OF THE INVENTION

The details of the present invention will now be described below.

The method for producing an L-lysine salt of biotin according to the present invention includes the following steps of:
(a) bringing crude biotin into contact with L-lysine in a solvent to obtain a solution or suspension of an L-lysine salt of biotin; and
(b) when the solution of the L-lysine salt of biotin is obtained in step (a), precipitating the L-lysine salt of biotin from the solution of the L-lysine salt of biotin.

It is noted that when the solution of the L-lysine salt of biotin is obtained in step (a), step (b) is performed, whereas when the suspension of the L-lysine salt of biotin is obtained in step (a), step (b) is not performed.

### (Crude biotin)

The crude biotin contains biotin and epibiotin. The crude biotin is typically a solid. In the crude biotin, the content (purity) of biotin in terms of HPLC area percentage is, in one embodiment, 90% or more and less than 99.92%. In the crude biotin, the content of epibiotin in terms of HPLC area percentage is, in one embodiment, about 5% or less.

The crude biotin can be a product obtained in the seventh step (stage 7) of the seven steps of biotin synthesis described above. That is, in the sixth step of the seven steps of biotin synthesis described above, when a vinyl sulfide (DVE) is reduced to N,N'-dibenzylbiotin (HVC), an epimer of N,N'-dibenzylbiotin is presumed to be produced as a by-product. Epibiotin is presumed to be produced through the deprotection reaction of this epimer of N,N'-dibenzylbiotin in the seventh step. The crude biotin may be a product obtained by subjecting the product obtained in the seventh step to a purification treatment such as an activated carbon treatment. The crude biotin may also be a commercially available biotin.

An example of the method for producing the crude biotin is as follows.

First, a vinyl sulfide (DVE) represented by the following formula (6) is synthesized from a ureide (URD) by a known method such as the method described in Patent Document 1.

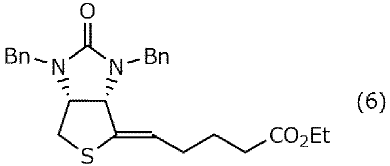

The vinyl sulfide (DVE) may be synthesized by the following method after synthesizing a lactone compound (LCT) represented by the following formula (4) by a known method such as the method described in Patent Document 1 (for example, Stage 1 to Stage 3 of Patent Document 1).

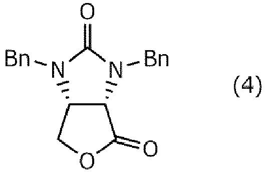

A mixture containing a thiolactone compound (DTL) represented by the following formula (5) is obtained by bringing the lactone compound (4) into contact with an alkali metal acetate and then reacting with a thiocarboxylic acid.

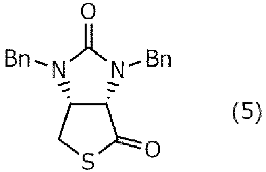

Under a nitrogen atmosphere, dihalogenoethane is added to a zinc powder to activate the zinc powder, and the activated zinc powder is then brought into contact with a halogenated alkane derivative to prepare an alkylzinc halide. The thiolactone compound (5) dissolved in an organic solvent is mixed with the alkylzinc halide, and an acid is added to the reaction solution to obtain the vinyl sulfide (DVE).

A product containing N,N'-dibenzylbiotin (HVC) represented by the following formula (7) is obtained by reducing the synthesized vinyl sulfide by a known method. This product contains an epimer of N,N'-dibenzylbiotin produced as a by-product.

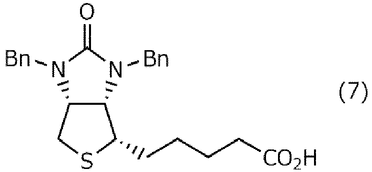

Mixing this product with an acid causes a debenzylation reaction of N,N'-dibenzylbiotin and its epimer to occur, whereby a product containing biotin and epibiotin is obtained. As the crude biotin, the product thus obtained may be used, or the product after being subjected to an activated charcoal treatment may be used.

The activated carbon treatment is a method of removing impurities from the crude biotin by mixing the crude biotin with activated carbon.

### (Biotin)

Biotin is represented by the following formula (1), and is also referred to as D-biotin.

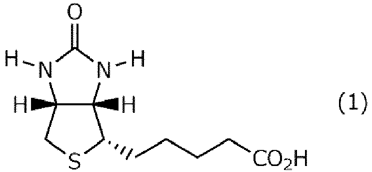

By bringing biotin into contact with L-lysine in a solvent, an L-lysine salt of biotin can be produced.

### (Epibiotin)

Epibiotin is represented by the following formula (3), and is also referred to as D-epibiotin. Epibiotin can contain L-epibiotin.

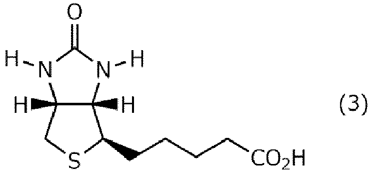

By bringing epibiotin into contact with L-lysine in a solvent, an L-lysine salt of epibiotin can be produced.

### (L-lysine)

L-lysine used in the present invention is not particularly limited.

The amount of L-lysine used with respect to 1 mol of biotin is preferably 0.50 mol or more and 2.0 mol or less, and more preferably 1.0 mol or more and 1.5 mol or less.

### (Solvent)

The solvent used for bringing crude biotin into contact with L-lysine preferably contains at least one selected from the group consisting of water, alcohol, acetone and acetonitrile. Among them, it is preferable to use water or an organic solvent composed of at least one selected from the group consisting of alcohol, acetone and acetonitrile, and it is more preferable to use water or alcohol. Water includes distilled water, purified water, pure water, ultrapure water, tap water or a mixture thereof. A water-containing organic solvent may also be used. The volume-based water content in the water-containing organic solvent is preferably 50% or less, and more preferably 20% or less.

The alcohol preferably contains at least one selected from the group consisting of methanol, ethanol and 2-propanol. Among those, it is preferable to use ethanol.

### (Production of L-lysine salt)

Examples of the method for producing an L-lysine salt of biotin according to the present invention include:
(1) a method for producing an L-lysine salt of biotin, including a step of bringing crude biotin into contact with L-lysine in water to obtain a solution of an L-lysine salt of biotin, and a step of precipitating the L-lysine salt of biotin from the solution of the L-lysine salt of biotin (hereinafter referred to as "production method (1)"); and
(2) a method for producing an L-lysine salt of biotin, including a step of bringing crude biotin into contact with L-lysine in an organic solvent containing at least one selected from the group consisting of alcohol, acetone and acetonitrile to obtain a suspension of an L-lysine salt of biotin (hereinafter referred to as "production method (2)").

According to each of production methods (1) and (2), a high-purity L-lysine salt of biotin with less epibiotin or a salt thereof can be obtained. In the obtained high-purity L-lysine salt of biotin, the content of epibiotin in terms of HPLC area percentage is, in one embodiment, 0.035% or less, in another embodiment, 0.020% or less, in yet another embodiment, 0.010% or less, and, in yet another embodiment, 0.004% or less. The lower limit is 0% or the detection limit. In the obtained high-purity L-lysine salt of biotin, the content (purity) of biotin in terms of HPLC area percentage is, in one embodiment, 99.92% or more, in another embodiment, 99.94% or more, in yet another embodiment, 99.95% or more, and, in yet another embodiment, 99.96% or more. The upper limit is 100%.

### (Production method (1))

### (Step of obtaining a solution of an L-lysine salt of biotin)

An L-lysine salt of biotin is represented by the following formula (8).

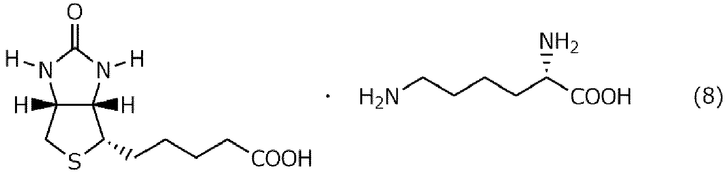

In the production method (1), first, a solution of an L-lysine salt of biotin is obtained by bringing crude biotin into contact with L-lysine in water. This step is one example of step (a).

The method of bringing the crude biotin into contact with the L-lysine in the water is not particularly limited. The solution of the L-lysine salt of biotin can be obtained by stirring and mixing the crude biotin and the L-lysine in the water. The addition order of the water, the crude biotin and the L-lysine is also not particularly limited.

In one embodiment, the amount of the water used with respect to 1 g of the crude biotin is, for example, 1 mL or more and 10 mL or less, and preferably 3 mL or more and 5 mL or less. In another embodiment, the amount of the water used with respect to 1 g of the crude biotin is, for example, 0.5 mL or more and 5 mL or less, and preferably 0.5 mL or more and 2 mL or less.

The temperature at which the crude biotin is brought into contact with the L-lysine is not particularly limited. The contact may be performed at room temperature, or at the temperature at which the solution of the L-lysine salt of biotin is brought into contact with a precipitation solvent in the next step (the step of precipitating the L-lysine salt of biotin).

After the crude biotin is brought into contact with the L-lysine in the water, the resulting mixture may be stirred and mixed. In the case of stirring, the stirring time is, for example, 1 minute or more and 24 hours or less, and preferably 10 minutes or more and 12 hours or less. The stirring may be performed at a temperature equal to or higher than the temperature at which the crude biotin is brought into contact with the L-lysine, or similarly to the above-described temperature at the time of contact, the stirring may be performed at room temperature, or at the temperature at which the solution of the L-lysine salt of biotin is brought into contact with a precipitation solvent in the next step.

The resulting L-lysine salt of biotin and L-lysine salt of epibiotin may each be in a dissolved state or solvated state in the water. In the present invention, the "solution of the L-lysine salt of biotin" is used in the sense that the "solution of the L-lysine salt of biotin" encompasses these states.

The fact that the L-lysine salt of biotin is produced in the solution of the L-lysine salt of biotin can be confirmed, for example, by nuclear magnetic resonance (NMR) spectroscopy.

### (Step of precipitating the L-lysine salt of biotin)

In production method (1), next, the L-lysine salt of biotin is precipitated from the solution of the L-lysine salt of biotin obtained in the above step (the step of obtaining a solution of an L-lysine salt of biotin) to obtain a precipitate of the L-lysine salt of biotin. This step corresponds to step (b).

The solution of the L-lysine salt of biotin may contain an L-lysine salt of epibiotin in addition to the L-lysine salt of biotin. Therefore, it is preferable to separate only the L-lysine salt of biotin from the solution of the L-lysine salt of biotin. Examples of the method for separating the L-lysine salt of biotin from the solution of the L-lysine salt of biotin include a method of mixing the solution of the L-lysine salt of biotin and a solvent for selectively dissolving or selectively precipitating the L-lysine salt of biotin. It is preferable to obtain a precipitate of the L-lysine salt of biotin by bringing the solution of the L-lysine salt of biotin into contact with the solvent for selectively precipitating the L-lysine salt of biotin (hereinafter also referred to as "precipitation solvent") to precipitate the L-lysine salt of biotin, from the viewpoint that the operation is easy.

As the precipitation solvent, it is preferable to use an organic solvent containing at least one selected from the group consisting of methanol, ethanol, 2-propanol, acetone and acetonitrile, and more preferable to use an organic solvent composed of at least one selected from the group consisting of methanol, ethanol, 2-propanol, acetone and acetonitrile. It is preferable to use as the precipitation solvent an organic solvent containing at least one selected from the group consisting of ethanol and 2-propanol, and more preferable to use as the precipitation solvent an organic solvent composed of at least one selected from the group consisting of ethanol and 2-propanol, from the viewpoint that their selectivity to the L-lysine salt of biotin is high. From the viewpoint of increasing the purity and yield of the L-lysine salt of biotin, it is more preferable to use 2-propanol.

In one embodiment, the amount of the precipitation solvent used with respect to 1 g of the crude biotin is, for example, 1 mL or more and 200 mL or less, preferably 10 mL or more and 100 mL or less, and more preferably 32 mL or more and 50 mL or less. In another embodiment, the amount of the precipitation solvent used with respect to 1 g of the crude biotin is, for example, 5 mL or more and 50 mL or less, and preferably 8 mL or more and 20 mL or less.

The volume ratio of the precipitation solvent to the water (precipitation solvent/water) is, for example, 1 or more and 50 or less, and preferably 5 or more and 30 or less. A higher volume ratio tends to increase the yield of the L-lysine salt of biotin. On the other hand, a smaller volume ratio tends to decrease the amount of epibiotin or a salt thereof contained in the L-lysine salt of biotin. Since it is possible to obtain a high-purity L-lysine salt of biotin with less epibiotin or a salt thereof, it is particular preferable to adjust the amount of the precipitation solvent used with respect to 1 g of the crude biotin and the volume ratio of the precipitation solvent to the water (precipitation solvent/water) to the above-described ranges. When two or more types of precipitation solvents are used as the precipitation solvent, the volume of the precipitation solvent means the total volume of the two or more types of precipitation solvents.

The temperature at which the solution of the L-lysine salt of biotin is brought into contact with the precipitation solvent is, for example, 0°C or higher and 100°C or lower, and preferably 40°C or higher and 80°C or lower. When the solution of the L-lysine salt of biotin is brought into contact with the precipitation solvent, the L-lysine salt of biotin is precipitated to form a suspension of the L-lysine salt of biotin. When the solution of the L-lysine salt of biotin is brought into contact with the precipitation solvent at a higher temperature, the fluidity of the solution of the L-lysine salt of biotin is increased, and the solution of the L-lysine salt of biotin is sufficiently brought into contact with the precipitation solvent. As a result, the amount of the L-lysine salt of biotin that is precipitated tends to increase, and thus the yield of the L-lysine salt of biotin tends to increase. It is noted that after the precipitation solvent is brought into contact with the solution of the L-lysine salt of biotin that has been heated or cooled to the above-described contact temperature, the resulting mixture may be further heated.

The contact between the solution of the L-lysine salt of biotin and the precipitation solvent can be performed by adding the precipitation solvent to the solution of the L-lysine salt of biotin. The precipitation solvent may be added to the solution of the L-lysine salt of biotin in multiple batches. For example, the precipitation solvent may be added dropwise while stirring. Alternatively, a first precipitation solvent may be added to the solution of the L-lysine salt of biotin and stirred for a certain period of time, and then a second precipitation solvent may be added to the mixture and stirred for a certain period of time. The first precipitation solvent and the second precipitation solvent may be of different types or may be of the same type. The amount of the first precipitation solvent and the amount of the second precipitation solvent may be the same or different.

When obtaining the suspension of the L-lysine salt of biotin, it is preferable to bring the solution of the L-lysine salt of biotin into contact with the precipitation solvent, and then stir and mix the resulting mixture. The stirring time is, for example, 1 minute or more and 24 hours or less, and preferably 10 minutes or more and 12 hours or less. The stirring is preferably performed at the temperature at the time of contact or higher, for example, at a temperature of 0°C or higher and 100°C or lower, and preferably 40°C or higher and 80°C or lower. If the mixture of the solution of the L-lysine salt of biotin and the precipitation solvent is stirred at a higher temperature, the fluidity of the mixture increases and the solution of the L-lysine salt of biotin is sufficiently brought into contact with the precipitation solvent. As a result, the amount of the L-lysine salt of biotin that is precipitated tends to increase, and thus the yield of the L-lysine salt of biotin tends to increase.

When obtaining the suspension of the L-lysine salt of biotin, it is preferred to stir and mix the mixture of the solution of the L-lysine salt of biotin and the precipitation solvent at the above temperature, then cool the mixture, and stir and mix the cooled mixture. The cooling temperature of the mixture of the solution of the L-lysine salt of biotin and the precipitation solvent may be, for example, -20°C or higher and 10°C or lower, and preferably -10°C or higher and 10°C or lower. When the mixture of the solution of the L-lysine salt of biotin and the precipitation solvent is cooled, the amount of the L-lysine salt of biotin that is precipitated tends to increase, and thus the yield of the L-lysine salt of biotin tends to increase. The stirring time is, for example, 1 minute or more and 24 hours or less, and preferably 10 minutes or more and 12 hours or less.

The precipitate of the L-lysine salt of biotin can be isolated from the suspension of the L-lysine salt of biotin by filtration or the like. It is then preferably washed with a washing solvent. As the washing solvent, the same type of solvent as the precipitation solvent is used. By drying the precipitate after washing, a solid of the L-lysine salt of biotin can be obtained. The L-lysine salt of biotin is, for example, in a powder state.

### (Production method (2))

In production method (2), a suspension of an L-lysine salt of biotin is obtained by bringing crude biotin into contact with L-lysine in an organic solvent containing at least one selected from the group consisting of alcohol, acetone and acetonitrile. This step is one example of step (a).

The method of bringing the crude biotin into contact with the L-lysine in the organic solvent is not particularly limited. It is sufficient if the crude biotin and the L-lysine are mixed in the organic solvent, and the addition order of the organic solvent, the crude biotin, and the L-lysine is not particularly limited.

The amount of the organic solvent used with respect to 1 g of the crude biotin is, for example, 1 mL or more, preferably 5 mL or more, and more preferably 8 mL or more. Since the L-lysine salt of biotin hardly dissolves in alcohol, the yield is not affected. Therefore, although there is no upper limit on the amount of the organic solvent used, in consideration of the operation properties, the amount used is preferably 100 mL or less, and more preferably 50 mL or less.

The organic solvent used for bringing the crude biotin into contact with the L-lysine is preferably an organic solvent composed of at least one selected from the group consisting of alcohol, acetone and acetonitrile, and more preferably alcohol. As described above, the alcohol preferably contains at least one selected from the group consisting of methanol, ethanol and 2-propanol, because this enables a high-purity L-lysine salt of biotin with less epibiotin or a salt thereof to be obtained. Among them, it is preferable to use ethanol.

Although the temperature at which the crude biotin is brought into contact with the L-lysine is not particularly limited, the temperature is, for example, 0°C or higher and 100°C or lower, and preferably 20°C or higher and 80°C or lower.

When the crude biotin is brought into contact with the L-lysine in the organic solvent, the crude biotin and the L-lysine gradually dissolve in the organic solvent, the produced L-lysine salt of biotin precipitates, and ultimately a suspension of the L-lysine salt of biotin is obtained.

After bringing the crude biotin into contact with the L-lysine in the organic solvent, the resulting mixture is preferably stirred and mixed. The stirring time is, for example, 1 minute or more and 24 hours or less, and preferably 30 minutes or more and 12 hours or less. The stirring is preferably performed at the temperature at the time of contact or higher, for example, at a temperature of 0°C or higher and 100°C or lower, and preferably 40°C or higher and 80°C or lower. When the mixture is stirred at a higher temperature, the fluidity of the mixture is increased, and biotin is sufficiently brought into contact with L-lysine. As a result, the amount of the L-lysine salt of biotin that is precipitated tends to increase, and thus the yield of the L-lysine salt of biotin tends to increase.

The obtained suspension of the L-lysine salt of biotin is preferably stirred and mixed at the above temperature, then cooled, and stirred and mixed. The cooling temperature of the suspension of the L-lysine salt of biotin may be, for example, -20°C or higher and 10°C or lower, and preferably -10°C or higher and 10°C or lower. When the suspension of the L-lysine salt of biotin is cooled, the amount of the L-lysine salt of biotin that is precipitated tends to increase, and thus the yield of the L-lysine salt of biotin tends to increase. The stirring time is, for example, 1 minute or more and 24 hours or less, and preferably 30 minutes or more and 12 hours or less.

The precipitate of the L-lysine salt of biotin can be isolated from the suspension of the L-lysine salt of biotin by filtration or the like. It is then preferably washed with a washing solvent. As the washing solvent, the same type of alcohol as that used when bringing the crude biotin into contact with the L-lysine is used. By drying the precipitate after washing, a solid of the L-lysine salt of biotin can be obtained. The L-lysine salt of biotin is, for example, in a powder state.

### (Method for producing biotin)

Biotin can be obtained by bringing an L-lysine salt of biotin obtained by the method for producing an L-lysine salt of biotin according to the present invention into contact with an acid to break down the L-lysine salt of biotin. Typically, a solid of biotin is precipitated by bringing the L-lysine salt of biotin into contact with the acid.

In the method for producing biotin according to the present invention, biotin is obtained by breaking down a high-purity L-lysine salt of biotin that hardly contains any epibiotin or a salt thereof. Thus, in the obtained biotin, the content of epibiotin in terms of HPLC area percentage can be very low. In the obtained biotin, the content of epibiotin in terms of HPLC area percentage is, in one embodiment, 0.1% or less, in another embodiment, 0.01% or less, and, in yet another embodiment, 0.006% or less. The lower limit of this content is 0% or the detection limit.

As the acid, for example, at least one acid selected from the group consisting of hydrochloric acid, sulfuric acid, methanesulfonic acid, phosphoric acid, sodium hydrogen phosphate, potassium hydrogen phosphate, and citric acid can be used. Hydrochloric acid is preferably used as the acid. The use of hydrochloric acid tends to reduce the amount of impurities such as epibiotin that can be contained in the L-lysine salt of biotin. The concentration of the hydrochloric acid may be, for example, 1% by mass or more and 20% by mass or less. Further, instead of an acid, an acid salt capable of forming the above-described acid may be used. As the acid salt, it is preferable to use at least one selected from the group consisting of potassium hydrogen sulfate, sodium hydrogen sulfate, sodium hydrogen phosphate, and potassium hydrogen phosphate. The acid and the acid salt may be used in combination.

The amount of the acid used with respect to 1 mol of the L-lysine salt of biotin is, for example, 0.1 mol or more and 20 mol or less, preferably 0.5 mol or more and 10 mol or less, and more preferably 1.0 mol or more and 3.0 mol or less.

The contact between the L-lysine salt of biotin and the acid may be performed after dissolving the L-lysine salt of biotin in the dissolving solvent. As a result of the contact with the acid, biotin is precipitated. The solvent for dissolving the L-lysine salt of biotin preferably includes at least one selected from the group consisting of water, methanol, ethanol, 2-propanol, acetone and acetonitrile. It is more preferable to use water as the dissolving solvent.

The amount of the dissolving solvent with respect to 1 g of the L-lysine salt of biotin is, for example, 1 mL or more and 100 mL or less, preferably 1 mL or more and 30 mL or less, and more preferably 1 mL or more and 15 mL or less.

After dissolving the L-lysine salt of biotin in the dissolving solvent, the contact with the acid is preferably performed while heating. The temperature at the time of contact is, for example, 50°C or higher and 120°C or lower, and preferably 70°C or higher and 100°C or lower.

The contact with the acid is preferably performed by adding the acid dropwise into a solution obtained by dissolving the L-lysine salt of biotin in the dissolution solvent. The time taken for dropping the acid is, for example, 10 minutes or more and 2 hours or less, and preferably 15 minutes or more and 2 hours or less.

It is preferable to add the acid while stirring the solution of the L-lysine salt of biotin to bring the solution of the L-lysine salt into contact with the acid, and then continue stirring at the temperature at the time of contact even after the addition of the acid. The stirring time after the addition of the acid is, for example, 1 minute or more and 5 hours or less, and preferably 20 minutes or more and 2 hours or less.

The mixed solution of the solution of the L-lysine salt of biotin and the acid is preferably stirred at the above temperature and then cooled. The cooling temperature of the mixed solution of the L-lysine salt of biotin and the acid is, for example, -20°C or higher and 10°C or lower, and preferably -10°C or higher and 10°C or lower. When the mixed solution of the solution of the L-lysine salt of biotin and the acid is cooled, the amount of biotin that is precipitated tends to increase, and thus the biotin yield tends to increase.

It is desirable to cool the mixed solution of the solution of the L-lysine salt of biotin and the acid while stirring. The cooling time of the mixed solution of the solution of the L-lysine salt of biotin and the acid is, for example, 1 minute or more and 5 hours or less, and preferably 20 minutes or more and 2 hours or less.

The precipitate of biotin can be isolated from the solution of the L-lysine salt of biotin to which the acid has been added by filtration or the like. The solid biotin separated from the L-lysine salt of biotin may then be washed with a washing solvent. As the washing solvent, the same dissolving solvent as the dissolving solvents that can dissolve the L-lysine salt of biotin can be used. High-purity biotin can be obtained by drying the washed solid at room temperature, for example.

The biotin thus obtained does not contain impurities such as epibiotin, and thus a very high purity can be achieved. In the obtained high-purity biotin, the content (purity) of biotin in terms of HPLC area percentage is, in one embodiment, 99.96% or more. The upper limit of the content (purity) in terms of HPLC area percentage is 100%.

In order to further increase the purity of the biotin obtained by the above method, the biotin may be further subjected to the purification treatment described above, such as a neutralization crystallization treatment or an activated carbon treatment, and a neutralization crystallization treatment with L-lysine may be repeated a plurality of times.

### EXAMPLES

The present invention will now be described in more detail with reference to the following examples, but these examples are not intended to limit the scope of the present invention.

### <Purity measurement>

In the crude biotin obtained in the production examples, the dried L-lysine salt of biotin obtained in the examples and the dried biotin obtained in the examples, the respective contents of biotin and epibiotin in terms of HPLC area percentage were measured using high performance liquid chromatography (HPLC) under the following conditions.

The content of biotin in terms of HPLC area percentage was determined as the ratio of the area value of the biotin peak with respect to the total area value of all peaks excluding the solvent peak and the L-lysine peak as measured under the following conditions. The content of epibiotin in terms of HPLC area percentage was determined in the same manner as above, except that the area value of the epibiotin peak was used instead of the area value of the biotin peak.

Apparatus: Waters Alliance (Registered Trademark) e2695, manufactured by Waters Corporation
Detector: UV absorption photometer Waters 2489
Measurement wavelength: 200 nm
Mobile phase A: 20 mM KH₂PO₄ aqueous solution (pH 3)
Mobile phase B: Acetonitrile
Mobile phase delivery:
   0 to 30 minutes: Mobile phase A 92%, mobile phase B 8%
   30 to 35 minutes: Mobile phase A 20%, mobile phase B 80%
   35 to 40 minutes: Mobile phase A 92%, mobile phase B 8%
Flow rate: 1.0 mL/min
Column temperature: 40°C
Column, filler: XBridge C18, 5 µm (4.6 x 150 mm)
RT of L-lysine: 1.493
RT of biotin: 11.912
RT of epibiotin: 15.729

### <Production Example 1 of crude biotin>

### (Synthesis of lactone compound (LCT))

First, 15.00 g of a lactone compound (LCT) was synthesized from a ureide (URD) by the method described in Patent Document 1 via three steps.

### (Synthesis of thiolactone compound (DTL))

15.00 g (0.047 mol) of the lactone compound, 7.31 g (0.074 mol) of potassium acetate, and 22.5 mL of N,N-dimethylacetamide (DMA) were placed in a 200 mL four-necked flask, and stirred at room temperature under a nitrogen flow to obtain a mixture.

The mixture was heated to a temperature of 125°C, then 4.25 g (0.056 mol) of thioacetic acid was added in 1/10 portions every 3 minutes. Stirring was performed at a temperature of 125°C for 2 hours to obtain a mixture containing a thiolactone compound.

Next, the mixture was cooled to a temperature of 80°C, and then 15 mL of DMA was added. 40 mL of water was further added over 30 minutes, and stirring was performed over 2 hours at a temperature of 25°C or more and 30°C or less to obtain a mixture containing a precipitate of the thiolactone compound. The mixture was filtered to separate the precipitate of the thiolactone compound by filtration. The separated precipitate of the thiolactone compound was washed with a washing liquid. A mixed solvent of 10 mL of acetone and 20 mL of water was used as the washing liquid. The amount of the precipitate of the thiolactone compound after washing was 16.34 g. When measured using HPLC under the conditions described above, the amount of the thiolactone compound contained in the thiolactone compound precipitate (wet) after washing was 13.39 g, and the yield from the lactone compound was 85% (stage 4).

### (Synthesis of ethyl zinc 5-iodovalerate)

Under a nitrogen atmosphere, a zinc powder (R powder manufactured by Hakusui Tech Co., Ltd., 4.29 g, 65.6 mmol) was suspended in a mixed solvent of tetrahydrofuran (THF, 7.0 mL) and toluene (4.9 mL), 1,2-dibromoethane (2.57 g, 13.7 mmol) was added dropwise at 75°C over 30 minutes, and the mixture was stirred at the same temperature for 30 minutes. Then, ethyl 5-iodovalerate (I-TAI, 7.0 g, 27.3 mmol) was added dropwise at 55°C over 30 minutes, and the mixture was stirred at the same temperature for 3 hours. A portion of the reaction solution was collected and analyzed by gas chromatography according to the method described above, from which the conversion rate to the zinc compound was shown to be 97.96%.

### (Synthesis of vinyl sulfide (DVE))

The reaction solution was cooled to 25°C, and a thiolactone compound (6.48 g, 19.1 mmol) and toluene (14 mL) were added. Then, a suspension of 10% Pd/C (179.4 mg, 0.169 mmol) in N,N-dimethylformamide (DMF, 1.8 mL) was added at the same temperature, and the mixture was stirred at 40°C for 3 hours and at 25°C for 14 hours.

After the reaction was completed, 16% hydrochloric acid (14 mL) was added at 25°C, the mixture was stirred at the same temperature for 1 hour, and the reaction solution was filtered. The filtrate was stirred at 40°C for 1 hour, then the organic layer was washed with water (14 mL, 2 x 21 mL), 5% aqueous sodium sulfite solution (21 mL), 5% aqueous sodium bicarbonate solution (21 mL), and water (14 mL), washed with water, and concentrated to obtain a vinyl sulfide. Analysis of the obtained organic layer by high performance liquid chromatography showed that the conversion rate to the vinyl sulfide was 98.37% (stage 5).

### (Synthesis of N,N'-dibenzylbiotin (HVC))

The vinyl sulfide (maximum 19.1 mmol) was dissolved in a mixed solution of methanol (59 mL) and water (16 mL), Pd(OH)₂/C (50% by mass (wet), 0.59 g) was added, and catalytic reduction was performed at a temperature of 11°C and a hydrogen pressure of 0.9 MPa for 12 hours. After the reaction was completed, the reaction solution was filtered, a 31% by mass NaOH aqueous solution (7.01 g) was added to the filtrate, and the mixture was stirred and mixed at 40°C for 2 hours. After hydrogenation was completed, a 10% by mass hydrochloric acid was added to the reaction solution to adjust the pH to 1. The methanol was distilled off under reduced pressure, and the product was extracted with ethyl acetate, washed with water and concentrated to obtain a product containing N,N'-dibenzylbiotin. This product contains an epimer of N,N'-dibenzylbiotin produced as a by-product.

### (Synthesis of biotin (BIF))

Mesitylene (6.6 mL) and sulfuric acid (1.7 mL) were added to N,N'-dibenzylbiotin (2.04 g, 4.81 mmol) at room temperature, and the mixture was stirred at 100°C for 2.5 hours. After the reaction was completed, the mesitylene was removed, toluene (7.7 mL) was added, and the mixture was stirred for 10 minutes. The toluene was removed, then water (25 mL) was divided into 8 portions and added at intervals of 10 minutes while maintaining the reaction solution at 80°C, and the mixture was stirred at the same temperature for 10 minutes. After allowing the reaction solution to cool, the reaction solution was cooled to 5°C and stirred for 3 hours. The obtained crystal was collected by filtration and washed with water (8.2 mL) and acetone (10 mL). Then, biotin (1.02 g, yield 86.5%) was obtained by drying under reduced pressure at 60°C for 16 hours. Hereinafter, this biotin is also referred to as crude biotin. In this crude biotin, the content (purity) of biotin in terms of HPLC area percentage was 99.914%, and the content of epibiotin in terms of HPLC area percentage was 0.037%.

### <Example 1>

### (Production of L-lysine salt of biotin BLS1)

1.0 g (4.09 mmol) of the crude biotin obtained in Production Example 1, 758 mg (4.50 mmol) of L-lysine, and 5 mL of distilled water were charged into a reaction vessel in that order, stirred, mixed, and dissolved to obtain a solution of an L-lysine salt of biotin. The solution was heated to 60°C with stirring, then 30 mL of 2-isopropanol (IPA) was added dropwise, and the mixture was stirred at the same temperature for 30 minutes. After the dropwise addition, the solution was cooled to 5°C with stirring, and then stirred overnight while maintaining the temperature at 5°C to precipitate an L-lysine salt of biotin. The precipitated L-lysine salt of biotin was collected by filtration and dried under reduced pressure at 25°C to obtain a dried L-lysine salt of biotin. The amount of the dried L-lysine salt of biotin was 1.32 g, and the yield was 82.67%. Hereinafter, this L-lysine salt of biotin is also referred to as L-lysine salt of biotin BLS1.

The results of 1H-NMR spectroscopic analysis of the obtained L-lysine salt of biotin BLS1 at 400 MHz are shown below. Heavy water (D₂O) was used as a solvent.
δ4.60 (m, 1H)
4.46 (m, 1H)
3.72 (t, 1H)
3.36 (m, 1H)
3.02 (m, 3H)
2.80 (d, 1H)
2.20 (t, 2H)
1.88 (m, 2H)
1.75 (m, 3H)
1.62 (m, 3H)
1.40-1.55 (m, 4H).

### <Example 2>

### (Production of L-lysine salt of biotin BLS2)

1.0 g (4.09 mmol) of the crude biotin obtained in Production Example 1, 758 mg (4.50 mmol) of L-lysine, and 5 mL of distilled water were charged into a reaction vessel in that order, stirred, mixed, and dissolved to obtain a solution of an L-lysine salt of biotin. The solution was heated to 60°C with stirring, then 40 mL of 2-isopropanol (IPA) was added dropwise, and the mixture was stirred at the same temperature for 30 minutes. After the dropwise addition, the solution was cooled to 5°C with stirring, and then stirred overnight while maintaining the temperature at 5°C to precipitate an L-lysine salt of biotin. The precipitated L-lysine salt of biotin was collected by filtration, and dried under reduced pressure at 25°C to obtain a dried L-lysine salt of biotin. The amount of the dried L-lysine salt of biotin was 1.58 g, and the yield was 98.67%. Hereinafter, this L-lysine salt of biotin is also referred to as L-lysine salt of biotin BLS2.

### <Example 3>

### (Production of L-lysine salt of biotin BLS3)

1.0 g (4.09 mmol) of the crude biotin obtained in Production Example 1, 758 mg (4.50 mmol) of L-lysine, and 5 mL of distilled water were charged into a reaction vessel in that order, stirred, mixed, and dissolved to obtain a solution of an L-lysine salt of biotin. The solution was heated to 60°C with stirring, then 50 mL of 2-isopropanol (IPA) was added dropwise, and the mixture was stirred at the same temperature for 30 minutes. After the dropwise addition, the solution was cooled to 5°C with stirring, and then stirred for 1 hour while maintaining the temperature at 5°C to precipitate an L-lysine salt of biotin. The precipitated L-lysine salt of biotin was collected by filtration, washed with 25 mL of 2-isopropanol, and dried under reduced pressure at 25°C to obtain a dried L-lysine salt of biotin. The amount of the dried L-lysine salt of biotin was 1.61 g, and the yield was quant. Hereinafter, this L-lysine salt of biotin is also referred to as L-lysine salt of biotin BLS3.

### <Example 4>

### (Production of L-lysine salt of biotin BLS4)

1.0 g (4.09 mmol) of the crude biotin obtained in Production Example 1, 758 mg (4.50 mmol) of L-lysine, and 4 mL of distilled water were charged into a reaction vessel in that order, stirred, mixed, and dissolved to obtain a solution of an L-lysine salt of biotin. The solution was heated to 60°C with stirring, then 32 mL of 2-isopropanol (IPA) was added dropwise, and the mixture was stirred at the same temperature for 30 minutes. After the dropwise addition, the solution was cooled to 5°C with stirring, and then stirred for 1 hour while maintaining the temperature at 5°C to precipitate an L-lysine salt of biotin. The precipitated L-lysine salt of biotin was collected by filtration, washed with 16 mL of 2-isopropanol, and dried under reduced pressure at 25°C to obtain a dried L-lysine salt of biotin. The amount of the dried L-lysine salt of biotin was 1.61 g, and the yield was quant. Hereinafter, this L-lysine salt of biotin is also referred to as L-lysine salt of biotin BLS4.

### <Example 5>

### (Production of L-lysine salt of biotin BLS5)

1.0 g (4.09 mmol) of the crude biotin obtained in Production Example 1, 758 mg (4.50 mmol) of L-lysine, and 1 mL of distilled water were charged into a reaction vessel in that order, stirred, mixed, and dissolved to obtain a solution of an L-lysine salt of biotin. The solution was heated to 60°C with stirring, then 20 mL of ethanol (EtOH) was added dropwise, and the mixture was stirred at the same temperature for 30 minutes. After the dropwise addition, the solution was cooled to 5°C with stirring, and then stirred for 1 hour while maintaining the temperature at 5°C to precipitate an L-lysine salt of biotin. The precipitated L-lysine salt of biotin was collected by filtration, washed with 10 mL of cold ethanol, and dried under reduced pressure at 25°C to obtain a dried L-lysine salt of biotin. The amount of the dried L-lysine salt of biotin was 1.58 g, and the yield was 98.60%. Hereinafter, this L-lysine salt of biotin is also referred to as L-lysine salt of biotin BLS5.

### <Example 6>

### (Production of L-lysine salt of biotin BLS6)

1.0 g (4.09 mmol) of the crude biotin obtained in Production Example 1, 758 mg (4.50 mmol) of L-lysine, and 0.5 mL of distilled water were charged into a reaction vessel in that order, stirred, mixed, and dissolved to obtain a solution of an L-lysine salt of biotin. The solution was heated to 60°C with stirring, then 10 mL of ethanol (EtOH) was added dropwise, and the mixture was stirred at the same temperature for 30 minutes. After the dropwise addition, the solution was cooled to 5°C with stirring, and then stirred for 1 hour while maintaining the temperature at 5°C to precipitate an L-lysine salt of biotin. The precipitated L-lysine salt of biotin was collected by filtration, washed with 25 mL of cold ethanol, and dried under reduced pressure at 25°C to obtain a dried L-lysine salt of biotin. The amount of the dried L-lysine salt of biotin was 1.61 g, and the yield was quant. Hereinafter, this L-lysine salt of biotin is also referred to as L-lysine salt of biotin BLS6.

### <Example 7>

### (Production of L-lysine salt of biotin BLS7)

5 g (20.47 mmol) of the crude biotin obtained in Production Example 1, 3.29 mg (20.51 mmol) of L-lysine, and 5 mL of distilled water were charged into a reaction vessel in that order, stirred, mixed, and dissolved to obtain a solution of an L-lysine salt of biotin. The solution was heated to 60°C with stirring, then 100 mL of ethanol (EtOH) was added dropwise, and the mixture was stirred at the same temperature for 30 minutes. After the dropwise addition, the solution was cooled to 5°C with stirring, and then stirred for 1 hour while maintaining the temperature at 5°C to precipitate an L-lysine salt of biotin. The precipitated L-lysine salt of biotin was collected by filtration, washed with 25 mL of cold ethanol, and dried under reduced pressure at 25°C to obtain a dried L-lysine salt of biotin. The amount of the dried L-lysine salt of biotin was 7.93 g, and the yield was 99.24 %. Hereinafter, this L-lysine salt of biotin is also referred to as L-lysine salt of biotin BLS7.

### <Example 8>

### (Production of L-lysine salt of biotin BLS8)

1.0 g (4.09 mmol) of the crude biotin obtained in Production Example 1, 758 mg (4.50 mmol) of L-lysine, and 20 mL of ethanol (EtOH) were charged into a reaction vessel in that order, stirred and mixed to obtain a suspension of an L-lysine salt of biotin. The suspension was heated to 60°C with stirring, then stirred at the same temperature for 30 minutes, then cooled to 5°C, and stirred for 1 hour at the same temperature. The L-lysine salt of biotin was collected by filtration, washed with 25 mL of ethanol at 25°C, and dried under reduced pressure at 25°C to obtain a dried L-lysine salt of biotin. The amount of the dried L-lysine salt of biotin was 1.61 g, and the yield was quant. Hereinafter, this L-lysine salt of biotin is also referred to as L-lysine salt of biotin BLS8.

### <Example 9>

### (Production of L-lysine salt of biotin BLS9)

1.0 g (4.09 mmol) of the crude biotin obtained in Production Example 1, 758 mg (4.50 mmol) of L-lysine, and 10 mL of ethanol (EtOH) were charged into a reaction vessel in that order, stirred and mixed to obtain a suspension of an L-lysine salt of biotin. The suspension was heated to 60°C with stirring, then stirred at the same temperature for 30 minutes, then cooled to 5°C, and stirred for 1 hour at the same temperature. The L-lysine salt of biotin was collected by filtration, washed with 15 mL of cold ethanol, and dried under reduced pressure at 25°C to obtain a dried L-lysine salt of biotin. The amount of the dried L-lysine salt of biotin was 1.61 g, and the yield was quant. Hereinafter, this L-lysine salt of biotin is also referred to as L-lysine salt of biotin BLS9.

### <Example 10>

### (Production of L-lysine salt of biotin BLS10)

1.0 g (4.09 mmol) of the crude biotin obtained in Production Example 1, 758 mg (4.50 mmol) of L-lysine, and 8 mL of ethanol (EtOH) were charged into a reaction vessel in that order, stirred and mixed to obtain a suspension of an L-lysine salt of biotin. The suspension was heated to 60°C with stirring, then stirred at the same temperature for 30 minutes, then cooled to 5°C, and stirred for 1 hour at the same temperature. The L-lysine salt of biotin was collected by filtration, washed with 20 mL of cold ethanol, and dried under reduced pressure at 25°C to obtain a dried L-lysine salt of biotin. The amount of the dried L-lysine salt of biotin was 1.62 g, and the yield was quant. Hereinafter, this L-lysine salt of biotin is also referred to as L-lysine salt of biotin BLS10.

### <Example 11>

### (Production of biotin)

6.0 g (15.37 mmol) of the dried L-lysine salt of biotin BLS7 was dissolved in 21.0 mL of distilled water to obtain a solution. The solution was heated to 90°C with stirring, and then 11.2 g (15.37 mmol) of 10% by mass hydrochloric acid was added dropwise over 30 minutes while maintaining the temperature at 90°C. After the dropwise addition of hydrochloric acid, the suspension was stirred at the same temperature for 30 minutes, cooled to 5°C with stirring, and then stirred at the same temperature for 30 minutes. The resulting precipitated biotin was collected by filtration and washed with 12 mL of distilled water. The washed biotin was dried under reduced pressure at 25°C to obtain dried biotin. The amount of dried biotin was 3.59 g, and the yield was 95.58%.

The conditions and measurement results for the production of the L-lysine salt of biotin in Examples 1 to 10 are summarized in Table 1 below. In Table 1, the "content" is the content (%) in terms of HPLC area percentage.

**[Table 1]**

| | L-Lysine Salt of Biotin | Used Crude Biotin (g) | Solvent | | Precipitation Solvent | | Dried L-Lysine Salt of Biotin | | |
|---|---|---|---|---|---|---|---|---|---|
| | | | Type | Amount (mL) | Type | Amount (mL) | Yield (%) | Biotin Content (%) | Epibiotin Content (%) |
| Example 1 | BLS1 | 1 | Water | 5 | IPA | 30 | 82.67 | 99.942 | 0.035 |
| Example 2 | BLS2 | 1 | Water | 5 | IPA | 40 | 98.67 | 99.961 | 0.006 |
| Example 3 | BLS3 | 1 | Water | 5 | IPA | 50 | quant. | 99.944 | 0.031 |
| Example 4 | BLS4 | 1 | Water | 4 | IPA | 32 | quant. | 99.962 | 0.004 |
| Example 5 | BLS5 | 1 | Water | 1 | EtOH | 20 | 98.60 | 99.962 | 0.004 |
| Example 6 | BLS6 | 1 | Water | 0.5 | EtOH | 10 | quant. | 99.950 | 0.020 |
| Example 7 | BLS7 | 5 | Water | 5 | EtOH | 100 | 99.24 | 99.966 | 0.006 |
| Example 8 | BLS8 | 1 | EtOH | 20 | - | - | quant. | 99.922 | 0.033 |
| Example 9 | BLS9 | 1 | EtOH | 10 | - | - | quant. | 99.923 | 0.033 |
| Example 10 | BLS10 | 1 | EtOH | 8 | - | - | quant. | 99.940 | 0.029 |

The conditions for the production of biotin in Example 11 and the measurement results for Production Example 1 and Example 11 are summarized in Table 2 below. In Table 2, the "content" is the content (%) in temrs of HPLC area percentage.

**[Table 2]**

| | L-Lysine Salt of Biotin | Solvent | Acid | Dried Biotin | | |
|---|---|---|---|---|---|---|
| | | Amount (mL) | Amount (mL) | Yield (%) | Biotin Content (%) | Epibiotin Content (%) |
| Production Example 1 | - | - | - | - | 99.914 | 0.037 |
| Example 11 | BLS 7 | 21.0 | 11.2 | 95.58 | 99.961 | 0.006 |

As shown in Table 1, by bringing crude biotin into contact with L-lysine in a solvent separating an L-lysine salt of biotin from a solution or suspension of the L-lysine salt of biotin, the L-lysine salt of biotin was obtained and the amount of epibiotin could be reduced. Further, as shown in Table 2, the dried biotin of Example 11, obtained by bringing the L-lysine salt of biotin into contact with an acid in a solvent, hardly contained any epibiotin, and the purity of the biotin could be increased.

## Claims

1. A method for producing an L-lysine salt of biotin, comprising the following steps of:
(a) bringing crude biotin into contact with L-lysine in a solvent to obtain a solution or suspension of an L-lysine salt of biotin; and
(b) when the solution of the L-lysine salt of biotin is obtained in step (a), precipitating the L-lysine salt of biotin from the solution of the L-lysine salt of biotin.

2. The method for producing an L-lysine salt of biotin as claimed in claim 1, wherein the solvent comprises at least one selected from the group consisting of water, methanol, ethanol, 2-propanol, acetone and acetonitrile.

3. The method for producing an L-lysine salt of biotin as claimed in claim 1 or 2, wherein step (a) is a step of bringing crude biotin into contact with L-lysine in water to obtain a solution of an L-lysine salt of biotin.

4. The method for producing an L-lysine salt of biotin as claimed in any one of claims 1 to 3, wherein step (b) is a step of precipitating the L-lysine salt of biotin by bringing the solution of the L-lysine of biotin into contact with a precipitation solvent.

5. The method for producing an L-lysine salt of biotin as claimed in claim 4, wherein the precipitation solvent comprises at least one selected from the group consisting of methanol, ethanol, 2-propanol, acetone and acetonitrile.

6. The method for producing an L-lysine salt of biotin as claimed in claim 4 or 5, wherein a volume ratio of the precipitation solvent to water (precipitation solvent/water) is 5 or more and 30 or less.

7. The method for producing an L-lysine salt of biotin as claimed in any one of claims 4 to 6, wherein step (b) is a step of precipitating the L-lysine salt of biotin by heating the solution of the L-lysine salt of biotin to 40°C or higher and 80°C or lower, then bringing the solution of the L-lysine salt of biotin into contact with the precipitation solvent, and then cooling the resulting mixture to -10°C or higher and 10°C or lower.

8. The method for producing an L-lysine salt of biotin as claimed in claim 1 or 2, wherein step (a) is a step of bringing crude biotin into contact with L-lysine in an organic solvent comprising at least one selected from the group consisting of alcohol, acetone and acetonitrile to obtain a suspension of an L-lysine salt of biotin.

9. The method for producing an L-lysine salt of biotin as claimed in claim 8, wherein the organic solvent is alcohol.

10. The method for producing an L-lysine salt of biotin as claimed in claim 8 or 9, wherein an amount of the organic solvent used is 8 mL or more with respect to 1 g of the crude biotin.

11. A method for producing biotin, comprising a step of producing an L-lysine salt of biotin by the method as claimed in any one of claims 1 to 10, and then bringing the L-lysine salt of biotin into contact with an acid, to obtain biotin.

12. An L-lysine salt of biotin.

13. The L-lysine salt of biotin as claimed in claim 12, wherein a content of epibiotin in terms of HPLC area percentage is 0.035% or less.
